# EUROPEAN PATENT APPLICATION

(11) **EP 1 795 603 A1**
(43) Date of publication of application: **13.06.2007**
(21) Application number: 05788164.1
(22) Date of filing: 27.09.2005
(51) Int. Cl.: C12P 17/16, C12P 17/18, C12Q 1/02, G01N 33/15, G01N 33/50, A61K 31/4523, C07D 405/06, C07D 493/04

(54) **PROCESS FOR SYNTHESIS OF DERIVATIVES, COMPOUND LIBRARY, METHOD FOR MAKING THE MASE, AND SCREENING METHOD**

(30) Priority: 27.09.2004 JP 2004279597
(71) Applicant: Keio University, Tokyo 108-8345 (JP)
(72) Inventor: IMOTO, Masaya, c/o Keio University, Yokohama-shi, Kanagawa 2238522 (JP); OHTA, Hiromichi, c/o Keio University, Yokohama-shi, Kanagawa 2238522 (JP); MIYAMOTO, Kenji c/o Keio University, Yokohama-shi, Kanagawa 2238522 (JP)
(74) Representative: Jones, Helen M.M.
(86) International application number: PCT/JP2005/017745
(87) International publication number: WO 2006/035770

(57) **Abstract**

[Object] To provide a method for synthesizing derivatives of natural compounds; a method for constructing a compound library containing derivatives of natural compounds; a compound library containing derivatives of natural compounds; and a screening method using a compound library, which are useful for a HTS random screening, a search for drugs or agricultural chemicals, or a search for a lead compound of a drug or an agricultural chemical.

[Solving Means] A derivative of an organic compound can be synthesized by culturing a microorganism producing the organic compound in a predetermined culture broth, and reacting the organic compound obtained by the culturing with a reaction reagent for synthesizing the derivative of the organic compound in the culturing broth. By constructing a library containing the thus obtained organic compound derivative, a HTS (high-throughput) random screening, a search for a drug or agricultural chemical, or a search for a lead compound of a drug or agricultural chemical can be performed.

## Description

### Cross-Reference To Related Documents

The present application claims the priority of a Japanese patent application No. 2004-279597 filed on September 27, 2004, which is incorporated herein by reference.

### Technical Field

The present invention relates to a method of synthesizing derivatives of organic compounds produced by a microorganism, a compound library containing the derivatives and a method of constructing the library, and a screening method using the compound library.

### Background Art

In the development of drugs and agricultural chemicals, commercially available compound libraries and compound libraries generated by combinatorial synthesis (for example, PCT Japanese Translation Patent Publication Nos. 2001-518053 and 2002-502393) have been screened (for example, PCT Japanese Translation Patent Publication Nos. 2002-514612, 2002-517474, and 2003-521673) for the purpose of searching for physiologically active substances such as lead compounds. However, useful compounds cannot be efficiently found in the present state.

On the other hand, a large number of drugs or lead compounds for drugs (for example, penicillin, tacrolimus (FK-506), and pravastatin) have been found from libraries of natural compounds isolated and purified from culture broths of microorganisms. Thus, natural compounds have a great diversity of activity and remarkable structures. Therefore, natural compounds libraries are thought to be crucially important in searching for lead compounds.

Further, it has been found that some derivatives obtained by chemically modified natural compounds have superior physiological activity, lower toxicity, and/or fewer adverse effects than the original natural compounds. Therefore, libraries of derivatives of natural compounds are also thought to be useful in efficiently searching for excellent drugs, and thus it is demanded to construct libraries containing derivatives of natural compounds.

### Summary of Invention

However, since the isolation and purification of natural compounds takes a long time and the finding of lead compounds useful as drugs is difficult, it has been difficult to construct libraries of natural compounds or derivatives thereof.

In addition, when a natural compound is isolated for the purpose of synthesizing a derivative of the natural compound for constructing a library, it is necessary to clarify the structure of the compound for specifying a method suitable for modifying the compound as well as to investigate reaction conditions for synthesizing the derivative. Thus, disadvantageously, it takes a long time to synthesize derivatives. Therefore, it is demanded to develop a method for rapidly synthesizing derivatives.

Consequently, it is an object of the present invention to provide a method for synthesizing derivatives of natural compounds, a method for constructing a compound library containing derivatives of natural compounds, a compound library containing derivatives of natural compounds, and a screening method using a compound library, which are useful for high-throughput (HTS) random screening, searching for drugs or agricultural chemicals, searching for lead compounds of drugs or agricultural chemicals, etc.

The present inventors have tried to construct libraries of natural compounds or derivatives thereof in order to solve the above-mentioned problems. Firstly, to culture broths used for culturing Streptomyces sp. MK929-43F1 which synthesizes a compound represented by Formula (1) below, either the Jones' reagent for oxidizing the compound or acetone/oxone monopersulfate for epoxidizing the compound was added as a reaction reagent. Then, ethyl acetate extracts from the culture broths were fractionated by high-performance liquid chromatography (HPLC). In addition, an extract from the culture broth without the addition of the reaction reagent was also fractionated by HPLC. By comparing separation patterns between them, substances which are contained in the extract from the culture broth which has been reacted with the reaction reagent but are not contained in the extract from the culture broth which has not been reacted with the reaction reagent were identified and recovered. As a result of structural analysis of these recovered substances, it has been found that an oxide (represented by Formula (2) below) of a compound represented by Formula (1) below can be obtained from the culture broth which has been reacted with the Jones' reagent, and an epoxidized compound (represented by Formula (3) below) of the compound represented by Formula (1) can be obtained from the culture broth which has been reacted with acetone/oxone monopersulfate.

From these results, it is revealed that a derivative of an organic compound produced by a microorganism can be rapidly obtained by adding a reaction reagent for synthesizing the derivative to a culture broth. Thus, the present inventors have completed the present invention.

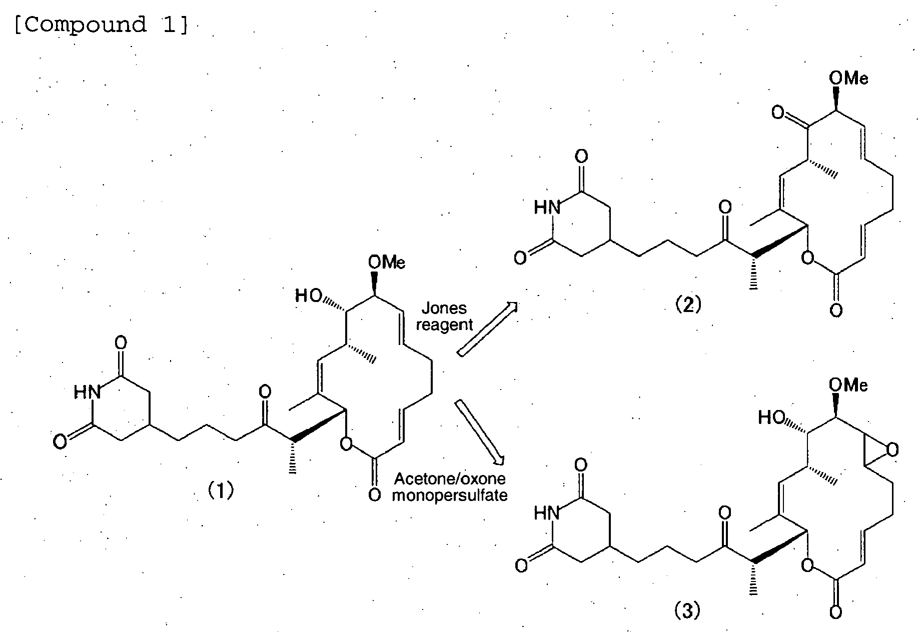

A method according to the present invention for synthesizing a derivative of an organic compound produced by a microorganism includes the steps of culturing the microorganism in a predetermined culture broth and reacting the organic compound obtained by culturing the microorganism with a reaction reagent for synthesizing the derivative of the organic compound in the culture broth. The reaction of the organic compound with the reaction reagent may be carried out by culturing the microorganism in the culture broth containing the reaction reagent, or may be carried out by producing the organic compound by culturing the microorganism in the culture broth substantially not containing the reaction reagent and adding the reaction reagent to the culture broth in which the microorganism has been cultured.

A method according to the present invention for constructing a compound library containing a derivative of an organic compound synthesized by reacting the organic compound produced by a microorganism with a reaction reagent includes steps of recovering a compound which is contained in a culture broth containing a reaction reagent and having been used for culturing the microorganism but is not contained in a culture broth substantially not containing the reaction reagent, and making the compound a member of the compound library.

A compound library according to the present invention containing a derivative of an organic compound synthesized by reacting the organic compound produced by a microorganism with a reaction reagent can be constructed by recovering a compound which is contained in a culture broth containing the reaction reagent and having been used for culturing the microorganism but is not contained in a culture broth substantially not containing the reaction reagent and making the compound a member of the compound library.

A screening method according to the present invention for a compound having a physiological activity using a compound library containing a derivative of an organic compound synthesized by reacting the organic compound produced by a microorganism with a reaction reagent includes a step of constructing the compound library by recovering a compound which is contained in a culture broth containing the reaction reagent and having been used for culturing the microorganism but is not contained in a culture broth substantially not containing the reaction reagent and making the compound a member of the compound library.

Further, a screening method according to the present invention for a therapeutic agent using a compound library containing a derivative of an organic compound synthesized by reacting the organic compound produced by a microorganism with a reaction reagent includes the steps of administering each compound in the compound library to a disease model animal other than human and evaluating whether or not the symptom of the disease is improved by the administration of the compound, wherein the compound library is constructed by recovering a compound which is contained in a culture broth containing the reaction reagent and having been used for culturing microorganism but is not contained in a culture broth substantially not containing the reaction reagent and making the compound a member of the compound library.

It should be noted that the above-mentioned culture broth substantially not containing the reaction reagent is a culture broth to be used for culturing the microorganism, and it may be the culture broth either before or after the culture of the microorganism. The recovery of the compound which is contained in a culture broth containing the reaction reagent and having been used for culturing the microorganism but is not contained in a culture broth substantially not containing the reaction reagent may be carried out by identifying the compound by fractionating compounds contained in the culture broth containing the reaction reagent and having been used for culturing the microorganism and also fractionating compounds contained in the culture broth substantially not containing the reaction reagent. The above-mentioned culture broth containing the reaction reagent and having been used for culturing the microorganism may be prepared by reacting the organic compound with the reaction reagent by culturing the microorganism in a culture broth containing the reaction reagent, or may be prepared by producing the organic compound by culturing the microorganism in a culture broth substantially not containing the reaction reagent and then adding the reaction reagent to the culture broth containing the organic compound produced by the microorganism for the reaction between the organic compound and the reaction reagent.

The above-mentioned microorganism may be, for example, a mutant carrying a mutation in a gene involved in a process of producing the organic compound, or a transformant generated by genetic manipulation of the gene involved in the process of producing the organic compound. The mutant carrying a mutation in the gene involved in the process of producing the organic compound may be a mutant carrying the mutation artificially introduced by, for example, ultraviolet irradiation, X-ray irradiation, or treatment with a chemical agent, or a spontaneous mutant.

The term "microorganism" in this description means a minute organism, and includes Archaea, Eubacteria, Archaezoa, Protozoa, Chromista, fungi (Eumycetes), and minute plants and animals. Archaea, also called archaeorganism or archaebacteria, includes extreme halophiles, thermophilic archaea, and methane bacteria (Methanogens). Eubacteria includes most bacteria such as colon bacillus and Actinomycetes. Archaezoa means Eukaryota which can live without a molecular enzyme (peroxisome) and includes trichomonas, enteromonas, oxymonas, microsporidian, naegleria, and diplomonas. Protozoa means mononuclear unicellular organism and includes Algae, Saprolegniaceae, Myxomycetes (slime molds), and cellular slime molds. Chromista means organisms characterized by that their chloroplast covered by two chloroplast envelopes is further covered with two envelopes, thus, covered with four envelopes in total, and includes Hyphochytridiomycetes, Oomycetes, and Labyrinthulomycetes. Fungi (Eumycetes) include Ascomycetes, Zygomycetes, Basidiomycetes, and Deuteromycetes and, for example, molds, mushrooms, and yeasts are included therein. Microorganisms included in the technical scope of the present invention are not limited to the organisms shown above, and any organisms are included as long as they can be treated similarly as the organisms shown above.

Examples of the above-mentioned reaction reagent include oxidizing reagents, reducing reagents, epoxidizing reagents, dihydroxylating reagents, oxidative cleavage reagents, hydrogenating reagents, etherifying reagents, halogenating reagents, nitrating reagents, sulfonating reagents, diazotizing reagents, aldol reaction reagents, and alkylating reagents, and one or more reagents selected from these reagents can be used as the reaction reagent. However, the reaction reagent is not limited to these reagents shown above.

The term "reaction reagent" used herein means a reagent which reacts with an organic compound produced by a microorganism to synthesize a derivative.

In particular, the "oxidizing reagents", "reducing reagents", "epoxidizing reagents", "dihydroxylating reagents", "etherifying reagents", "halogenating reagents", "nitrating reagents", "sulfonating reagents", "diazotizing reagents", and "alkylating reagents" denote reagents which, respectively, oxidize, reduce, epoxidize, dihydroxylate, etherify, halogenate, nitrate, sulfonate, diazotize, and alkylate a reaction substrate such as an organic compound produced by a microorganism.

The "oxidative cleavage reagents" denote reagents which oxidize and cleave an organic compound. The "hydrogenating reagents" denote reagents which substitute a functional group of an organic compound produced by a microorganism with hydrogen, or add hydrogen to an organic compound produced by a microorganism. The "aldol reaction reagents" denote reagents which nucleophilically add a ketone, aldehyde or ester having the same or different structure to that of an organic compound produced by a microorganism to generate an aldol or a compound readily induced from an aldol.

It should be noted that any reagents having the above-mentioned function can be used. For example, the reagent may contain a compound which directly reacts with an organic compound as a target, a compound having a catalytic activity, or the both. Brief Description of thr Drawings

FIG. 1 shows separation patterns of the extract of a culture broth of Streptomyces sp. MK929-43F1 to which the Jones' reagent or acetone/oxone monopersulfate have not been added (upper), the extract of a culture broth of Streptomyces sp. MK929-43F1 to which the Jones' reagent has been added (middle), and the extract of a culture broth of Streptomyces sp. MK929-43F1 to which acetone/oxone monopersulfate have been added (lower), in one embodiment of the present invention.

FIG. 2 shows a result of ¹H-NMR structure analysis of substances contained in the extract of a culture broth of Streptomyces sp. MK929-43F1 to which the Jones' reagent has been added in one embodiment of the present invention.

FIG. 3 shows a result of ¹H-NMR structure analysis of substances contained in the extract of a culture broth of Streptomyces sp. MK929-43F1 to which acetone/oxone monopersulfate has been added in one embodiment of the present invention.

FIG. 4 shows results of observation on activities of migrastatin and oxidized migrastatin on cell migration inhibitory in one embodiment of the present invention.

FIG. 5 shows results of observation on effects of migrastatin and oxidized migrastatin on ATP synthesis in one embodiment of the present invention.

### Best Mode for Carrying Out the Invention

The embodiments of the present invention completed on the basis of the above-mentioned finding will now be described in detail with reference to examples. Methods disclosed in standard protocols such as J. Sambrook, E. F. Fritsch & T. Maniatis (Ed.), Molecular cloning, a laboratory manual (3rd edition). Cold Spring Harbor Press, Cold Spring Harbor, New York (2001); F. M. Ausubel, R. Brent, R. E. Kingston, D. D. Moore, J. G. Seidman, J. A. Smith, K. Struhl (Ed.), Current Protocols in Molecular Biology, John Wiley & Sons Ltd. , or modification or alteration thereof are used, unless a specific description is given to the embodiments or examples. When a commercially available reagent kit or measurement apparatus is used, the kit and apparatus are used according to protocols attached thereto unless a specific description is given.

It should be noted that objects, features, advantages, and concepts of the present invention are obvious to those skilled in the art by the disclosure in the description, and the present invention can be readily practiced by those skilled in the art in accordance with the disclosure in the description. The embodiments of the present invention and specific examples described below are merely preferable specific examples for illustrating or describing the present invention and the present invention is not limited thereto. It is obvious to those skilled in the art that various alteration and modification can be added thereto based on the disclosure in the description within the concept and scope of the present invention disclosed in the description.

Firstly, for the purpose of synthesizing a target derivative of an organic compound, microorganism is cultured in a predetermined culture broth, and the reaction of an organic compound obtained by the culture of the microorganism with a reaction reagent for synthesizing the derivative of the organic compound is carried out in the culture broth.

The microorganism to be used in the present invention is not limited as long as the microorganism produces an organic compound as a metabolic product under culturing conditions, and may be any of fungi (such as molds, mushrooms, and yeasts, in general), bacteria (prokaryotic unicellular organisms), and Myxomycetes (slime molds). Genus or species of the microorganism is not limited, and examples of the microorganism include Eumycetes such as Ascomycetes (yeast, neurospora, penicillium, aspergillus, cup fungus, truffle, and the like), Zygomycetes (mucor, pilobolus, and the like), Basidiomycetes (matsutake, tree jellyfish, and the like), Deuteromycetes (botrytis and the like), and Chytridiomycetes; bacteria such as Eubacteria (colon bacillus, actinomycetes, and the like) and Archaea; and Myxomycetes (stemonitales and the like). Further, a mutant strain carrying a mutation in a gene involved in a process of producing an organic compound, as well as a wild-type strain, may be used to provide diversity to the organic compounds produced by the microorganism. The mutant strain may be a mutant carrying a mutation artificially introduced thereinto with ultraviolet, X-ray, or a chemical agent, or may be a transformant generated by genetic manipulation of the gene involved in the process of producing an organic compound, or may be a spontaneous mutant.

The culture of these microorganisms may be carried out according to a culture method generally used for the respective microorganism. Any culture broths allowing the microorganism to grow may be used for the culture, examples of which include synthetic culture media, semi-synthetic culture media, and natural culture media. Nutrients known as nutritional sources for the microorganism may be added to the culture broth. Examples of carbon sources include carbohydrates such as commercially available syrup, glucose, maltose, fructose, mannitol, potato starch, cornstarch, dextrin, and soluble starches; and fats and oils. Examples of nitrogen sources include inorganic or organic nitrogen sources such as commercially available peptones, meat extract, corn steep liquor, cottonseed meal, peanut powder, soybean powder, yeast extracts, NZ-amine, wheat germ, caseins, fish flour, sodium nitrate, and ammonium nitrate. A metal salt, such as sulfate, hydrochloride, nitrate, phosphate, or carbonate of Na, K, Mg, Ca, Zn, Fe, Mn, Co, or Cu, may be added according to need. Furthermore, an amino acid such as valine, leucine, isoleucine, phenylalanine, tryptophan, methionine, lysine, arginine, glutamic acid, or aspartic acid; or a secondary metabolite production-promoting agent, or antifoaming agent such as a vitamin, oleic acid, methyl oleate, lard oil, silicon oil, or surfactant, may be optionally used, according to need. Further, in addition to these additives, any other additives which can be utilized by microorganism and help the production of a secondary metabolite may be used. The culture may be carried out as the same manner as that of microorganism for manufacturing secondary metabolites. The method of the culture may be solid culture or liquid culture. The liquid culture may be static culture, shaking culture, or stirring culture.

Under such culturing conditions, an organic compound is produced as a secondary metabolite by culturing the microorganism. A derivative of the organic compound can be obtained by reacting the organic compound with a reaction reagent for synthesizing an organic compound derivative in the culture broth. There is a significantly high probability that the thus obtained derivative is a novel compound having a unique structure. Further, the derivative can be expected to have an activity which is not present naturally.

Examples of types of reaction and reaction reagent used in the reaction are listed below. Any types of reaction and reaction reagent can be employed as long as they can modify a functional group or skeleton of the organic compound obtained as a secondary metabolite, and the types of reactions and reaction reagents are not limited to those listed below. In addition, a combination of two or more of the individual reactions may be employed. Oxidizing reaction (reagent)
1) Oxidation of alcohol (CrO₃-H₂SO₄ (Jones' reagent), KMnO₄, Na₂CrO₇, NaOCl-TEMPO etc.)
2) Oxidation at benzyl-position or allyl-position (CrO₃AcOH, O₂/catalyst etc.)
3) Baeyer-Villiger reaction (peroxide and the like)
   Reducing reaction (reagent)
4) Reduction of carbonyl compound (aldehyde or ketone) (NaBH₄, NaBH₃CN, LiAlH₄, H₂/catalyst (for example, Rh, Ru, or Pd) etc.)
   Epoxidizing reaction (reagent)
5) Epoxidation of olefin (peracid (peracetic acid, m-chloroperbenzoic acid etc.), H₂O₂/catalyst, oxone-acetone etc.)
   Hydroxylating reaction (reagent)
6) Dihydroxylation of olefin (OsO₄, KMnO₄, R-CO₃H/H⁺ etc.)
   Oxidative cleavage reaction (reagent)
7) Oxidative cleavage of olefin (O₃, NaIO₄ etc.)
   Hydrogenating reaction (reagent)
8) Hydrogenation of olefin (H₂/Pd or Pt, Raney Ni etc.)
   Etherifying reaction (reagent)
9) Etherification of alcohol ((CH₃)₂SO₄, ICH₂CO₂H, N-ethylmaleimide)
   Halogenating reaction (reagent)
10) Halogenation of aromatic ring or olefin (F₂, XeF₂, Cl₂, Br₂, I₂, HCl, HBr, HOCl)
   Nitrating reaction (reagent)
11) Nitration of aromatic ring (HNO₃/H₂SO₄)
   Sulfonating reaction (reagent)
12) Sulfonation of aromatic ring (SO₃/H₂SO₄)
   Diazotizing reaction (reagent)
13) Diazotization of amine (NaNO₂/H⁺)
   Aldol reaction (reagent)
14) Aldol reaction (Lewis acid/silyl enol ether)
   Alkylating reaction (reagent)
15) Alkylation of ketone (R-MgX, RLi)
16) Alkylation of α-position of carbonyl (base/R-X)

The reaction between any of these reaction reagents and the organic compound obtained as a secondary metabolite in a culture broth may be carried out either by culturing the microorganism in a culture broth which has been added with the reaction reagent or by culturing microorganism in a culture broth substantially not containing the reaction reagent and then adding the reaction reagent to the culture broth after the culture of the microorganism. In the latter method, namely, when the reaction reagent is added to the broth after the culturing, the synthesis reaction may be carried out, for example, by directly adding the reagent for synthesis to the culture broth of the microorganism, or by extracting a culture broth containing the microorganism or a culture filtrate obtained by centrifugation or filtration of the culture broth to which a filter aid has been added, with an organic solvent which is not miscible with water, such as ethyl acetate, chloroform, benzene, toluene, or ether, and then adding the reagent for synthesis to the extract.

A compound library can be constructed by synthesizing derivatives of an organic compound produced by a microorganism by reacting the organic compound with various reaction reagents as described above and recovering the derivatives. For example, a library containing concentrated derivatives of an organic compound can be constructed by identifying and recovering compounds which are contained in a culture broth containing the reaction reagent and used for culturing the microorganism but are not contained in a culture broth substantially not containing the reaction reagent. In particular, a library can contain not only the derivatives of the organic compound produced by the microorganism but also the organic compound itself by using a culture broth before the culturing of the microorganism as the culture broth substantially not containing the reaction reagent. In addition, derivatives of an organic compound mainly produced by a microorganism can be identified and a library containing such derivatives at high frequency can be constructed by using a cultured broth which has been used for culturing the microorganism but not added with the reaction reagent as the culture broth substantially not containing the reaction reagent.

Methods for identifying and isolating an organic compound produced by a microorganism and/or a derivative of the organic compound may be any of common methods, such as column chromatography using silica gel, ODS, or Toyopearl HW-40, centrifugal liquid-liquid partition chromatography, thin layer chromatography, and high-performance liquid chromatography (HPLC). By utilizing any of these methods, compounds contained in a culture broth containing the reaction reagent and having been used for culturing the microorganism are fractionated, and similarly compounds contained in a culture broth substantially not containing the reaction reagent are fractionated. By comparing these fraction patterns, organic compounds and/or derivatives of organic compounds to be recovered can be readily identified and isolated.

The recovered compounds may be used as a mixture or may be used separately according to purpose. For example, if the identification of a compound having a physiological activity requires troublesome assays for individual compounds, the following process may be employed. Firstly, a library is divided into some pools, and a pool having the activity is identified. Then, compounds contained in the pool are divided into some sub-pools. Further, a sub-pool having the activity is identified similarly. By repeating these steps, a target compound can be identified by a smaller number of times of assays.

The structures of compounds constituting the library can be determined by any of known methods for structural analysis such as mass spectrometry, multiple mass spectrometry, UV/visible absorption spectrometry, proton nuclear magnetic resonance spectrometry, carbon-13 nuclear magnetic resonance spectrometry, infrared absorption spectrometry, and X-ray crystal spectrometry, or a combination thereof. The compounds, after their structures have been thus determined, may be dried under reduced pressure and stored in a cool dark place, e.g., in a refrigerator.

Furthermore, for the purpose of obtaining a compound having a physiological activity, various screenings can be carried out using the library. Examples of the compound having a physiological activity include, but not limited to, enzyme inhibitors, ligand/receptor binding inhibitors, angiogenesis inhibitors, cell adhesion inhibitors, gene expression inhibitors, and growth factor-like active substances. Examples of the enzyme inhibitors include tyrosine kinase inhibitors, cyclooxygenase (COX) inhibitors, telomerase inhibitors, matrix metalloprotease inhibitors, prostaglandin D synthesis inhibitors, phosphodiesterase inhibitors, cholinesterase inhibitors, virus protease inhibitors, and reverse transcriptase inhibitors. Examples of the receptor include adrenaline receptors, histamine receptors, leukotriene receptors, and opioid receptors.

### Examples

The present invention will now be described further in detail with reference to examples and drawings. In the examples, nuclear magnetic resonance spectra (¹H-NMR and ¹³C-NMR) were measured using JNM-AL300 (manufactured by JEOL Ltd.). Each reaction was carried out in argon unless a specific description is given.

### [Example 1]

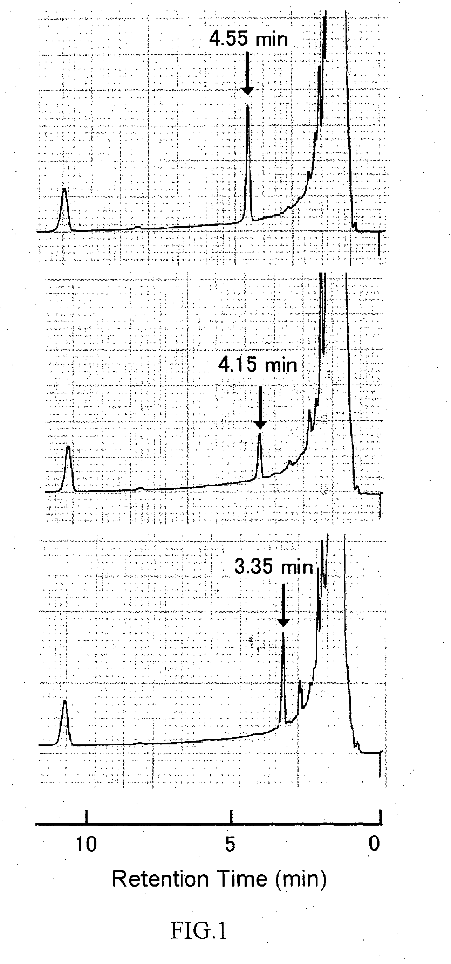

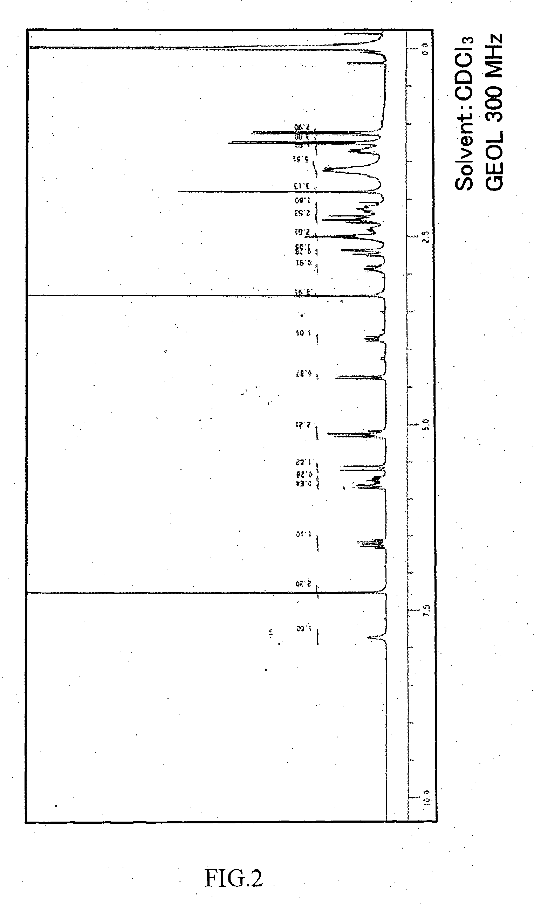

Streptomyces sp. MK929-43F1, which synthesizes a compound (migrastatin) represented by the above-mentioned Formula (1), was cultured in a culture broth (2% dextrin, 2% glycerol, 1% soy peptone, 0.3% yeast extract, 0.2% ammonium sulfate, 0.2% calcium carbonate, pH 7.4) at 27°C for 4 days. Then, a supernatant was obtained by centrifugation. To 60 µl of the obtained culture supernatant, 30 µl of 164 mg/ml the Jones' reagent was added, and the resulting mixture was stirred at 25°C for 1 min. In addition, a filtrate obtained from a culture broth not added with the Jones' reagent was also prepared as a control. After the stirring, 180 µl of a saturated sodium hydrogencarbonate aqueous solution was added to the mixture for neutralization, and then extraction was carried out with 720 µl of ethyl acetate. Then, the extract was concentrated and dried under reduced pressure, and the dried residue was dissolved in 300 µl of methanol. The obtained crude product was separated by high-performance liquid chromatography (column; SenshuPAK ODS C18 150 mm × 4.6φ, elution system; acetonitrile : water = 50 : 50) and a separation pattern (refer to FIG. 1) was obtained by a UV detector (manufactured by Shimadzu, UV: 220 nm). By comparing the separation pattern with that of the control, a substance which was contained in the extract from the cultured broth to which the Jones' reagent was added but was not contained in the extract from the cultured broth to which the Jones' reagent was not added was identified and recovered. The structure of this substance was analyzed by NMR (refer to FIG. 2) to confirm that the substance was an oxide (compound represented by the above-mentioned Formula (2): migrastatin oxide) of the compound represented by the Formula (1). Thus, an oxide of an organic compound could be obtained by using the Jones' reagent.

### [Example 2]

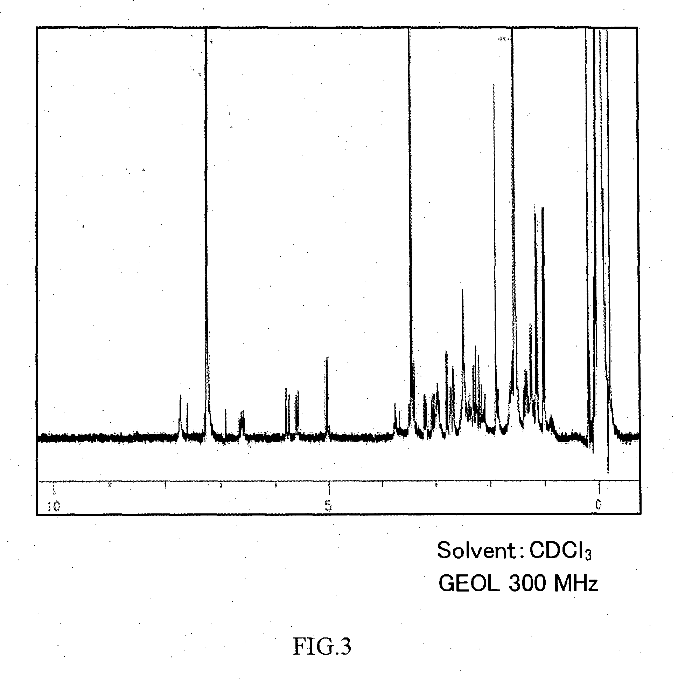

The filtrate (60 µl) obtained in Example 1 was extracted with 720 µl of ethyl acetate, and the extract was concentrated and dried under reduced pressure, and the dried residue was dissolved in 100 µl of acetone. Then, 1 mg of NaHCO₃ was added thereto for saturation. Further, 100 µl of 20 mg/ml oxone monopersulfate (dissolved in acetone) was added thereto, and the resulting mixture was stirred at room temperature for 3 hr. In addition, a solution without the addition of acetone/oxone monopersulfate was also prepared as a control. After the stirring, the extraction with 600 µl of ethyl acetate was carried out, and the extract was concentrated and dried under reduced pressure, and the dried residue was dissolved in 300 µl of methanol. The obtained crude product was separated by high-performance liquid chromatography (column: SenshuPAK ODS C18 150 mm × 4.6φ, elution system; acetonitrile : water = 50 : 50) and separation pattern (refer to FIG. 1) was obtained by a UV detector (manufactured by Shimadzu, UV: 220 nm). By comparing the separation pattern with that of the control, a substance which was contained in the extract from the cultured broth to which acetone/oxone monopersulfate was added but was not contained in the extract from the cultured broth to which acetone/oxone monopersulfate was not added was identified and recovered. The structure of this substance was analyzed by NMR (refer to FIG. 3) to confirm that the substance was an epoxidized compound (compound represented by the above-mentioned Formula (3): epoxy migrastatin) of the compound represented by the Formula (1). Thus, an epoxidized compound of an organic compound could be obtained by using acetone/oxone monopersulfate.

### [Example 3]

Migrastatin has been known to inhibit migration of a cancer cell. Thus, the following experiment was conducted in order to examine whether the oxidized migrastatin obtained in Example 1 can inhibit tumor cell migration or not.

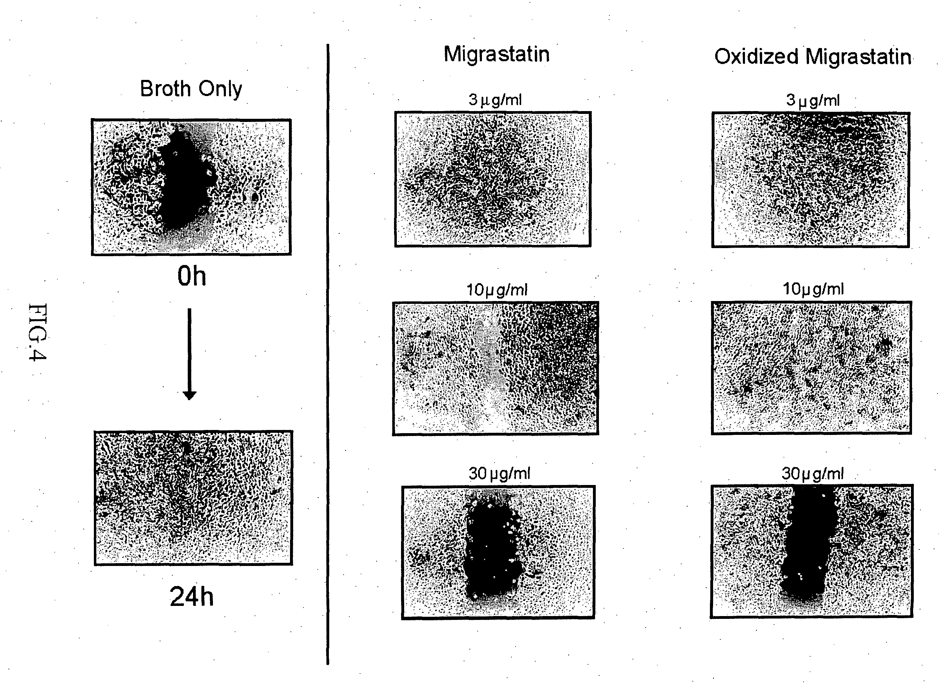

500 µl of suspension of EC17 cells derived from human esophageal cancer (1.5 × 10⁵ cells/ml; RPMI1640 medium (Nissui)) was added to each well of a 48-well plate and incubated at 37°C for 24 hr. Then, the cells were linearly scraped off by scratching the center of the bottom of each well in a straight line with a micropipette tip. Then, the supernatant of the culture broth was immediately removed. The well was washed with 300 µl of PBS⁻ (8 g/l NaCl, 0.2 g/l KCl, 0.916 g/l Na₂HPO₄, 0.2 g/l KH₂PO₄) carefully not to scrape the remaining cells, and 500 µl of a RPMI1640 medium containing 1% serum (FBS; manufactured by Tissue Culture Biologicals) was gently added to the well. Further, migrastatin or oxidized migrastatin was added thereto and incubated at 37°C for 24 hr. In addition, a culture broth to which migrastatin and oxidized migrastatin were not added was similarly incubated as a control. After the incubation, how much the straight line formed with the micropipette tip was filled with cells which had migrated from surroundings was confirmed by microscopic observation to evaluate migration of the cells. FIG. 4 shows the results.

As shown in FIG. 4, in the control, the line formed by scratching EC17 cells on the culture plate (0 hr) was filled with cells after the incubation of 24 hr by the migration of the cells. However, in the case where migrastatin was added to the culture broth immediately after the scratching (0 hr), it was observed that the migration of cells was inhibited depending on the concentration of migrastatin and was completely inhibited at a concentration of 30 µl/ml. Similarly, in the case where oxidized migrastatin was added to the culture broth immediately after the scratching, the inhibition effect was observed at almost the same concentration range.

In summary, it was revealed that oxidized migrastatin, which is one of derivatives of migrastatin, could inhibit migration of tumor cells as much as migrastatin does. In addition, it was revealed that a derivative of a compound may have the same activity as the original compound.

### [Example 4]

Next, the following experiment was conducted in order to confirm whether or not oxidized migrastatin can suppress ATP synthesis as much as migrastatin does.

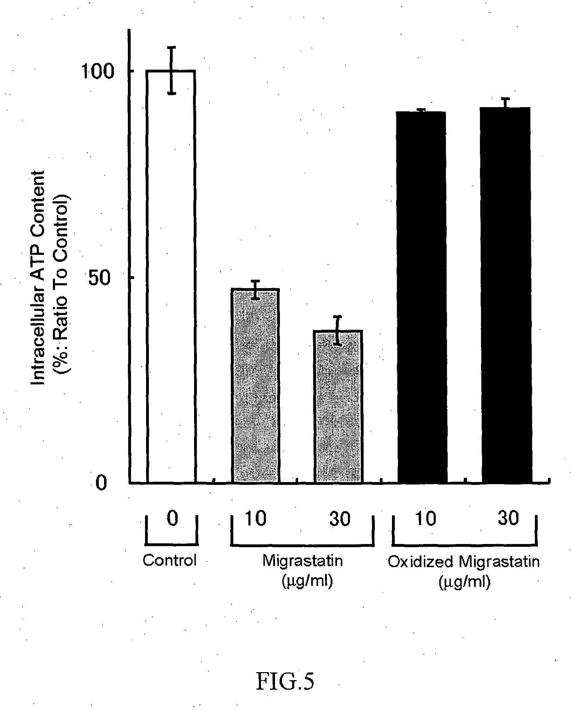

1 ml of suspension of HT-29 cells derived from human colon cancer (4 × 10⁵ cells/ml; RPMI1640 medium) was added to each well of a 24-well plate and incubated at 37°C for 24 hr. Then, migrastatin or oxidized migrastatin was added thereto and incubated for 3 hr. In addition, a cultured broth to which migrastatin and oxidized migrastatin were not added was similarly incubated as a control. After the incubation, each well was washed twice with ice-cooled PBS⁻. After an addition of 200 µl of 2% trichloroacetic acid, the plate was left at 4°C for 30 min. The supernatant (160 µl) was neutralized with 36.8 µl of 0.5 N NaOH and used as a sample. This sample (160 µl) was added to 3 ml of PBS⁻ containing 4 mM MgCl₂. Then, 40 µl of 4 mg/ml luciferase luciferin (Sigma) was added thereto and immediately the ATP content was measured with a single-photon monitor of a scintillation counter (LS-5000TD: BECKMAN COULTER). In addition, the ATP content under the conditions where the sample and luciferase luciferin were not added was measured as a blank value and was subtracted from each measurement value. FIG. 5 shows the results.

As shown in FIG. 5, it was revealed that migrastatin inhibited ATP synthesis in the same concentration range as it inhibits the cell migration, whereas oxidized migrastatin did not inhibit ATP synthesis at all in the same concentration range. Thus, it was confirmed that the ATP synthesis-inhibiting effect was lost by oxidizing the hydroxyl group of migrastatin. This suggests that the hydroxyl group of migrastatin is important for inhibiting ATP synthesis. In summary, it was revealed that a derivative of a compound may not have the same activity as the original compound. This suggests that the screening method according to the present invention is useful for obtaining a compound having a different activity, for example, identifying a compound not causing adverse effects.

### Industrial Applicability

According to the present invention, a method for synthesizing derivatives of substances produced by microorganisms, a method for constructing a library containing derivatives of substances produced by microorganisms, a compound library containing derivatives of substances produced by microorganisms, and a screening method using the compound library, which are useful for high-throughput (HTS) random screening, searching for drugs or agricultural chemicals, searching for lead compounds of drugs or agricultural chemicals, etc., can be provided.

## Claims

1. A method for synthesizing a derivative of an organic compound produced by a microorganism, comprising the steps of:
culturing the microorganism using a predetermined culture broth; and
reacting the organic compound obtained by culturing the microorganism with a reaction reagent for synthesizing the derivative of the organic compound in the culture broth.

2. The method according to claim 1, wherein the organic compound is reacted with the reaction reagent by culturing the microorganism in the culture broth containing the reaction reagent.

3. The method according to claim 1, wherein the organic compound is produced by culturing the microorganism in the culture broth substantially not containing the reaction reagent; and
the organic compound is reacted with the reaction reagent by adding the reaction reagent to the culture broth having been used for culturing the microorganism.

4. The method according to any one of claims 1 to 3, wherein the microorganism is a mutant carrying a mutation in a gene involved in a process of producing the organic compound.

5. The method according to claim 4, wherein the microorganism is a mutant carrying a mutation artificially introduced by ultraviolet irradiation, X-ray irradiation, or treatment with a chemical agent.

6. The method according to claim 4, wherein the microorganism is a spontaneous mutant.

7. The method according to any one of claims 1 to 3, wherein the microorganism is a transformant generated by genetic manipulation of a gene involved in a process of producing the organic compound.

8. The method according to any one of claims 1 to 7, wherein the microorganism belongs to any one selected from the group consisting of Archaea, Eubacteria, Protista, and Fungi.

9. The method according to any one of claims 1 to 7, wherein the microorganism belongs to any one selected from the group consisting of Ascomycetes, Zygomycetes, Basidiomycetes, Deuteromycetes, Myxomycectes, and Acrasiomycetes.

10. The method according to any one of claims 1 to 7, wherein the microorganism is actinomycete.

11. The method according to any one of claims 1 to 10, wherein the reaction reagent is one or more reagents selected from the group consisting of oxidizing reagents, reducing reagents, epoxidizing reagents, dihydroxylating reagents, oxidative cleavage reagents, hydrogenating reagents, etherifying reagents, halogenating reagents, nitrating reagents, sulfonating reagents, diazotizing reagents, aldol reaction reagents, and alkylating reagents.

12. A method for constructing a compound library containing a derivative of an organic compound synthesized by reacting the organic compound produced by a microorganism with a reaction reagent, comprising the steps of:
recovering a compound which is contained in a culture broth containing the reaction reagent and having been used for culturing the microorganism but is not contained in a culture broth substantially not containing the reaction reagent; and
making the compound a member of the compound library.

13. The method according to claim 12, wherein the culture broth substantially not containing the reaction reagent is a culture broth having been used for culturing the microorganism.

14. The method according to claim 12 or 13, wherein the compound which is contained in a culture broth containing the reaction reagent and having been used for culturing the microorganism but is not contained in a culture broth substantially not containing the reaction reagent is identified and recovered, by fractionating compounds contained in the culture broth containing the reaction reagent and having been used for culturing the microorganism and fractionating compounds contained in the culture broth substantially not containing the reaction reagent.

15. The method according to any one of claims 12 to 14, wherein the culture broth containing the reaction reagent and having been used for culturing the microorganism is obtained by reacting the organic compound with the reaction reagent by culturing the microorganism in a culture broth containing the reaction reagent.

16. The method according to any one of claims 12 to 14, wherein the culture broth containing the reaction reagent and having been used for culturing the microorganism is obtained by producing the organic compound by culturing the microorganism in a culture broth substantially not containing the reaction reagent and reacting the organic compound with the reaction reagent by adding the reaction reagent to the culture broth containing the organic compound produced by the microorganism.

17. The method according to any one of claims 12 to 16, wherein the microorganism is a mutant carrying a mutation in a gene involved in a process of producing the organic compound.

18. The method according to claim 17, wherein the microorganism is a mutant carrying a mutation artificially introduced by ultraviolet irradiation, X-ray irradiation, or treatment with a chemical agent.

19. The method according to claim 17, wherein the microorganism is a spontaneous mutant.

20. The method according to any one of claims 12 to 16, wherein the microorganism is a transformant generated by genetic manipulation of a gene involved in a process of producing the organic compound.

21. The method according to any one of claims 12 to 20, wherein the microorganism belongs to any one selected from the group consisting of Archaea, Eubacteria, Protista, and Fungi.

22. The method according to any one of claims 12 to 20, wherein the microorganism belongs to any one selected from the group consisting of Ascomycetes, Zygomycetes, Basidiomycetes, Deuteromycetes, Myxomycectes, and Acrasiomycetes.

23. The method according to any one of claims 12 to 20, wherein the microorganism is actinomycete.

24. The method according to any one of claims 12 to 23, wherein the reaction reagent is one or more reagents selected from the group consisting of oxidizing reagents, reducing reagents, epoxidizing reagents, dihydroxylating reagents, oxidative cleavage reagents, hydrogenating reagents, etherifying reagents, halogenating reagents, nitrating reagents, sulfonating reagents, diazotizing reagents, aldol reaction reagents, and alkylating reagents.

25. A compound library containing a derivative of an organic compound synthesized by reacting the organic compound produced by a microorganism with a reaction reagent,
being constructed by recovering a compound which is contained in a culture broth containing the reaction reagent and having been used for culturing the microorganism but is not contained in a culture broth substantially not containing the reaction reagent and making the compound a member of the compound library.

26. The compound library according to claim 25, wherein the culture broth substantially not containing the reaction reagent is a culture broth having been used for culturing the microorganism.

27. The compound library according to claim 25 or 26, wherein the compound which is contained in a culture broth containing the reaction reagent and having been used for culturing the microorganism but is not contained in a culture broth substantially not containing the reaction reagent is identified and recovered by fractionating compounds contained in the culture broth containing the reaction reagent and having been used for culturing the microorganism and also fractionating compounds contained in the culture broth substantially not containing the reaction reagent.

28. The compound library according to any one of claims 25 to 27, wherein the culture broth containing the reaction reagent and having been used for culturing the microorganism is obtained by reacting the organic compound with the reaction reagent by culturing the microorganism in a culture broth containing the reaction reagent.

29. The compound library according to any one of claims 25 to 27, wherein the culture broth containing the reaction reagent and having been used for culturing the microorganism is obtained by producing the organic compound by culturing the microorganism in a culture broth substantially not containing the reaction reagent and
reacting the organic compound with the reaction reagent by adding the reaction reagent to the culture broth containing the organic compound produced by the microorganism.

30. The compound library according to any one of claims 25 to 29, wherein the microorganism is a mutant carrying a mutation in a gene involved in a process of producing the organic compound.

31. The compound library according to claim 30, wherein the microorganism is a mutant carrying a mutation artificially introduced by ultraviolet irradiation, X-ray irradiation, or treatment with a chemical agent.

32. The compound library according to claim 30, wherein the microorganism is a spontaneous mutant.

33. The compound library according to any one of claims 25 to 29, wherein the microorganism is a transformant generated by genetic manipulation of a gene involved in a process of producing the organic compound.

34. The compound library according to any one of claims 25 to 33, wherein the microorganism belongs to any one selected from the group consisting of Archaea, Eubacteria, Protista, and Fungi.

35. The compound library according to any one of claims 25 to 33, wherein the microorganism belongs to any one selected from the group consisting of Ascomycetes, Zygomycetes, Basidiomycetes, Deuteromycetes, Myxomycectes, and Acrasiomycetes.

36. The compound library according to any one of claims 25 to 33, wherein the microorganism is actinomycete.

37. The compound library according to any one of claims 25 to 36, wherein the reaction reagent is one or more reagents selected from the group consisting of oxidizing reagents, reducing reagents, epoxidizing reagents, dihydroxylating reagents, oxidative cleavage reagents, hydrogenating reagents, etherifying reagents, halogenating reagents, nitrating reagents, sulfonating reagents, diazotizing reagents, aldol reaction reagents, and alkylating reagents.

38. A method for screening for a compound having a physiological activity using a compound library containing a derivative of an organic compound synthesized by reacting the organic compound produced by a microorganism with a reaction reagent, comprising steps of:
constructing the compound library by recovering a compound which is contained in a culture broth containing the reaction reagent and having been used for culturing the microorganism but is not contained in a culture broth substantially not containing the reaction reagent; and
making the compound a member of the compound library.

39. The screening method according to claim 38, wherein the culture broth substantially not containing the reaction reagent is a culture broth having been used for culturing the microorganism.

40. The screening method according to claim 38 or 39, wherein the compound which is contained in a culture broth containing the reaction reagent and having been used for culturing the microorganism but is not contained in a culture broth substantially not containing the reaction reagent is identified and recovered by fractionating compounds contained in the culture broth containing the reaction reagent and having been used for culturing the microorganism and fractionating compounds contained in the culture broth substantially not containing the reaction reagent.

41. The screening method according to any one of claims 38 to 40, wherein the culture broth containing the reaction reagent and having been used for culturing the microorganism is obtained by reacting the organic compound with the reaction reagent by culturing the microorganism in a culture broth containing the reaction reagent.

42. The screening method according to any one of claims 38 to 40, wherein the culture broth containing the reaction reagent and having been used for culturing the microorganism is obtained by producing the organic compound by culturing the microorganism in a culture broth substantially not containing the reaction reagent and
reacting the organic compound with the reaction reagent by adding the reaction reagent to the culture broth containing the organic compound produced by the microorganism.

43. The screening method according to any one of claims 38 to 42, wherein the microorganism is a mutant carrying a mutation in a gene involved in a process of producing the organic compound.

44. The screening method according to claim 43, wherein the microorganism is a mutant carrying a mutation artificially introduced by ultraviolet irradiation, X-ray irradiation, or treatment with a chemical agent.

45. The screening method according to claim 43, wherein the microorganism is a spontaneous mutant.

46. The screening method according to any one of claims 38 to 42, wherein the microorganism is a transformant generated by genetic manipulation of a gene involved in a process of producing the organic compound.

47. The screening method according to any one of claims 38 to 46, wherein the microorganism belongs to any one selected from the group consisting of Archaea, Eubacteria, Protista, and Fungi.

48. The screening method according to any one of claims 38 to 46, wherein the microorganism belongs to any one selected from the group consisting of Ascomycetes, Zygomycetes, Basidiomycetes, Deuteromycetes, Myxomycectes, and Acrasiomycetes.

49. The screening method according to any one of claims 38 to 46, wherein the microorganism is actinomycete.

50. The screening method according to any one of claims 38 to 49, wherein the reaction reagent is one or more reagents selected from the group consisting of oxidizing reagents, reducing reagents, epoxidizing reagents, dihydroxylating reagents, oxidative cleavage reagents, hydrogenating reagents, etherifying reagents, halogenating reagents, nitrating reagents, sulfonating reagents, diazotizing reagents, aldol reaction reagents, and alkylating reagents.

51. A method for screening for a therapeutic agent using a compound library containing a derivative of an organic compound synthesized by reacting the organic compound produced by a microorganism with a reaction reagent, comprising the steps of:
administering each compound in the compound library to a disease model animal other than human, the compound library being constructed by recovering a compound which is contained in a culture broth containing the reaction reagent and having been used for culturing the microorganism but is not contained in a culture broth substantially not containing the reaction reagent and making the compound a member of the compound library; and
evaluating whether or not the symptom of the disease is improved by the administration of the compound..

52. The screening method according to claim 51, wherein the culture broth substantially not containing the reaction reagent is a culture broth having been used for culturing the microorganism.

53. The screening method according to claim 51 or 52, wherein the compound which is contained in a culture broth containing the reaction reagent and having been used for culturing the microorganism but is not contained in a culture broth substantially not containing the reaction reagent is identified and recovered by fractionating compounds contained in the culture broth containing the reaction reagent and having been used for culturing the microorganism and fractionating compounds contained in the culture broth substantially not containing the reaction reagent.

54. The screening method according to any one of claims 51 to 53, wherein the culture broth containing the reaction reagent and having been used for culturing the microorganism is obtained by reacting the organic compound with the reaction reagent by culturing the microorganism in a culture broth containing the reaction reagent.

55. The screening method according to any one of claims 51 to 53, wherein the culture broth containing the reaction reagent and having been used for culturing the microorganism is obtained by producing the organic compound by culturing the microorganism in a culture broth substantially not containing the reaction reagent and
reacting the organic compound with the reaction reagent by adding the reaction reagent to the culture broth containing the organic compound produced by the microorganism.

56. The screening method according to any one of claims 51 to 55, wherein the microorganism is a mutant carrying a mutation in a gene involved in a process of producing the organic compound.

57. The screening method according to claim 56, wherein the microorganism is a mutant carrying a mutation artificially introduced by ultraviolet irradiation, X-ray irradiation, or treatment with a chemical agent.

58. The screening method according to claim 56, wherein the microorganism is a spontaneous mutant.

59. The screening method according to any one of claims 51 to 55, wherein the microorganism is a transformant generated by genetic manipulation of a gene involved in a process of producing the organic compound.

60. The screening method according to any one of claims 51 to 59, wherein the microorganism belongs to any one selected from the group consisting of Archaea, Eubacteria, Protista, and Fungi.

61. The screening method according to any one of claims 51 to 59, wherein the microorganism belongs to any one selected from the group consisting of Ascomycetes, Zygomycetes, Basidiomycetes, Deuteromycetes, Myxomycectes, and Acrasiomycetes.

62. The screening method according to any one of claims 51 to 59, wherein the microorganism is actinomycete.

63. The screening method according to any one of claims 51 to 62, wherein the reaction reagent is one or more reagents selected from the group consisting of oxidizing reagents, reducing reagents, epoxidizing reagents, dihydroxylating reagents, oxidative cleavage reagents, hydrogenating reagents, etherifying reagents, halogenating reagents, nitrating reagents, sulfonating reagents, diazotizing reagents, aldol reaction reagents, and alkylating reagents.
